# EUROPÄISCHE PATENTSCHRIFT

(11) **EP 0 574 736 B1**
(45) Veröffentlichungstag und Bekanntmachung des Hinweises auf die Patenterteilung: **17.07.1996**
(21) Anmeldenummer: 93108384.4
(22) Anmeldetag: 25.05.1993
(51) Int. Cl.: A61B 17/16

(54) **Schleif-Bohrwerkzeug für Knochenmaterial**
Drilling and abrasion tool for bony materials
Outil de perçage et de meulage de matériaux osseux

(30) Priorität: 06.06.1992 DE 4218722
(43) Veröffentlichungstag der Anmeldung: 22.12.1993
(73) Patentinhaber: MERCK PATENT GmbH, D-64271 Darmstadt (DE)
(72) Erfinder: Bauer, Hans-Jörg, W-6509 Flomborn (DE)

(56) Entgegenhaltungen:
- WO-A-83/02553
- FR-A- 2 625 429
- GB-A- 2 140 336
- US-A- 5 055 105

## Beschreibung

Die Erfindung betrifft ein Schleif-Bohrwerkzeug zur Gewinnung von Knochenmaterial durch rotierendes Fräsen, bestehend aus einem hohlzylindrischen Werkzeugschaft, der an seinem vorderen Ende einen ringförmigen, an seiner Oberfläche mit Schleifkornmaterial besetzten Schleifkopf trägt.

Derartige Schleifbohrwerkzeuge dienen in der medizinischen Technik zur Vorbereitung der Entnahme von Knochenmaterial und zur Vorbereitung eines Implantatbettes.

Gegenüber den mit definierten Werkzeugschneiden ausgestatteten bekannten Bohr- oder Fräswerkzeugen, die erhebliche Verletzungen an Weichteilen hervorrufen, wenn sie versehentlich mit benachbartem Weichteilgewebe in Berührung kommen, haben die nach dem Schleifverfahren arbeitenden Werkzeuge den Vorteil, daß sie bei der Berührung mit Weichteilgewebe keine Zerreißungen oder ähnliche Verletzungen verursachen.

Bei einem bekannten Schleif-Bohrwerkzeug der eingangs genannten Gattung, gemäß EP-PS 99 371, die zur Bildung des Oberbegriffs des Anspruchs 1 herangezogen worden ist, ist die Stirnfläche des Schleifkopfes abgerundet. Dies führt insbesondere beim Eindringen in Weichteilgewebe oder Knorpel, aber auch beim Eindringen in Knochen zu einem seitlichen Auswandern des Schleifkopfes, sofern nicht eine sehr genaue und stabile Führung des Werkzeugs erfolgt.

Aufgabe der Erfindung ist es daher, ein Schleif-Bohrwerkzeug der eingangs genannten Gattung so auszubilden, daß sich der Schleifkopf beim Eindringen in verhältnismäßig weiches Material selbst seitlich führt, so daß ein seitliches Auswandern des Schleifkopfes wirksam verhindert wird.

Diese Aufgabe wird erfindungsgemäß dadurch gelöst, daß die Stirnfläche des Schleifkopfes an ihrem inneren und/oder äußeren Rand scharfkantig an die benachbarte innere bzw. äußere zylindrische Seitenwand des Schleifkopes anschließt.

Diese scharfkantige Ausführung mindestens eines der Ränder der ringförmigen Stirnfläche führt dazu, daß an diesem Rand unabhängig von der Härte und Beschaffenheit des Gewebes, in das das Schleif-Bohrwerkzeug eindringt, ein genau begrenzter Schnitt erfolgt, so daß der Schleifkopf auch bei Knorpel und ähnlichem Gewebe eine seitliche Führung erhält, die ein seitliches Auswandern des Schleifkopfes verhindert. Gleichwohl wird ein Zerreißen oder eine sonstige Beschädigung des benachbarten Weichteilgewebes verhindert, weil bei dem Schleifvorgang nur die sehr feinen Schneiden der einzelnen Schleifkörner zum Eingriff kommen.

Gemäß einer bevorzugten Ausführungsform der Erfindung ist die Stirnfläche des Schleifkopfes eine senkrecht zur Werkzeuglängsachse verlaufende Kreisringfläche. Vorzugsweise ist diese Stirnfläche an ihren beiden Rändern scharfkantig ausgeführt. Dadurch erfolgt ein sauberer Trennvorgang sowohl am äußeren wie auch am inneren Rand der Stirnfläche des Schleifkopfes, während die mit Schleifkornmaterial besetzte Stirnfläche selbst den gesamten Materialabtrag übernimmt.

Gemäß anderen vorteilhaften Ausgestaltungen des Erfindungsgedankens kann die Stirnfläche des Schleifkopfes eine ringförmige äußere Kegelstumpffläche sein, die an ihrem inneren Rand einen scharfkantigen spitzen Winkel mit der inneren Seitenwand des Schleifkopfes bildet oder sie kann eine innere ringförmige Hohlkegelstumpf-Fläche sein, die an ihrem äußeren Rand einen scharfkantigen spitzen Winkel mit der äußeren Seitenwand des Schleifkopfes bildet. Durch die Kegelform der Stirnfläche wird erreicht, daß jeweils die eine Kante mit der benachbarten Seitenfläche einen spitzen Winkel bildet und deshalb besonders gut für einen sauberen Trennvorgang auch beim Eindringen in Knorpel und ähnliches Material geeignet ist.

Vorteilhafterweise ist der Schleifkopf an seinen der Stirnfläche abgekehrten rückwärtigen Ende gegenüber der Außenwand und der Innenwand des hohlzylindrischen Werkzeugschaftes jeweils mit einem Überstand von mindestens etwa 10 % der Wanddicke des Werkzeugschaftes ausgeführt.

Dieser rückwärtige Überstand des Schleifkopfes verhindert, daß die Wand des bearbeiteten Knochen- oder Knorpelmaterials mit dem Werkzeugschaft in stärkere Berührung kommt. Gegenüber einer beim bekannten Stand der Technik vorgeschlagenen konischen Ausführung des Werkzeugschaftes wird durch die Vermeidung bzw. Verminderung der Berührung zwischen dem Knochen bzw. Knorpel und den hohlzylindrischen Werkzeugschaft verhindert, daß das Werkzeug abgebremst und das Gewebe im Berührungsbereich durch Reibung erhitzt wird. Außerdem wird eine Unterbrechung der Strömung des Kühlmittels verhindert, das üblicherweise im Inneren des Werkzeugschaftes zugeführt wird.

Weitere vorteilhafte Ausgestaltungen des Erfindungsgeankens sind Gegenstand weiterer Unteransprüche, die insbesondere die Anordnung von Schlitzen, Nuten oder Bohrungen zur Verbesserung der Kühlmittelströmung im Bereich des Schleifkopfes betreffen.

Nachfolgend werden Ausführungsbeispiele der Erfindung näher erläutert, die in der Zeichnung dargestellt sind. Jeweils in einem Längsschnitt zeigt:
Fig. 1 ein Schleif-Bohrwerkzeug für Knochenmaterial mit kreisringförmiger Stirnfläche,
Fig. 2 den vorderen Abschnitt eines Schleif-Bohrwerkzeugs ähnlich der Fig. 1, jedoch mit einer Ausführung der Stirnfläche als innere ringförmige Hohlkegelstumpf-Fläche,
Fig. 3 ein Schleif-Bohrwerkzeug ähnlich der Fig. 1, wobei die Stirnfläche des Schleifkopfes jedoch eine äußere ringförmige Kegelstumpffläche ist,
Fig. 4 ein Schleif-Bohrwerkzeug ähnlich der Fig. 1, jedoch mit Kühlmittelschlitzen,
Fig. 5 ein Schleif-Bohrwerkzeug ähnlich der Fig. 2, jedoch mit Kühlmittelbohrungen und
Fig. 6 ein Schleif-Bohrwerkzeug ähnlich der Fig. 1, jedoch mit äußeren oder inneren Kühlmittelnuten.

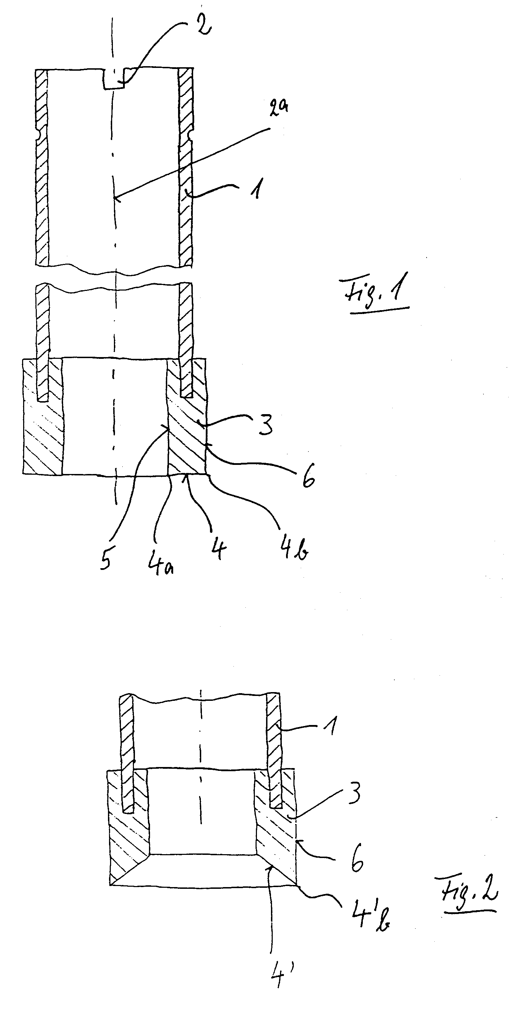

Das in Fig. 1 dargestellte Schleif-Bohrwerkzeug dient zur Gewinnung von Knochenmaterial in der medizinischen Technik, insbesondere zur Vorbereitung der Entnahme von Knochenmaterial und der Vorbereitung von Knochenimplantaten. Das Schleif-Bohrwerkzeug ist zur Aufnahme in ein (nicht dargestelltes) Spannfutter einer in der medizinischen Technik gebräuchlichen Bohrmaschine bestimmt. Ein hohlzylindrischer Werkzeugschaft 1 weist an seinem hinteren Ende eine Mitnehmernut 2 auf, in die ein (nicht dargestellter) Mitnehmer des Spannfutters der Bohrmaschine greift. Im Inneren des Werkzeugschaftes 1 wird üblicherweise Kühlmittel zugeführt.

Der Werkzeugschaft 1 trägt an seinem vorderen Ende einen ringförmigen Schleifkopf 3, der vorzugsweise ebenfalls hohlzylindrisch ausgeführt ist. Der Schleifkopf ist an seiner Oberfläche mit Schleifkornmaterial besetzt, üblicherweise Diamantkörnern oder Korundkörner. Der so gebildete Belag bildet das Schleifmaterial, das den Knochen oder Knorpel abträgt, in den das Schleif-Bohrwerkzeug eindringt. Der Schleifkopf 3 kann auch durchgehend aus den Schleifkornmaterial bestehen.

Der Schleifkopf 3 weist beim Ausführungsbeispiel nach Fig. 1 eine plane Stirnfläche 4 auf, die senkrecht zur Werkzeuglängsachse 2a verläuft und an ihrem inneren Rand 4a und ihrem äußeren Rand 4b scharfkantig an die benachbarte innere zylindrische Seitenwand 5 bzw. äußere zylindrische Seitenwand 6 des Schleifkopfes 3 anschließt. Diese scharfkantig begrenzte Ausführung der Stirnfläche 4 bewirkt, daß der Schleifkopf 3 in das abzutragende Knochen- bzw. Knorpelmaterial mit einem sauber begrenzten Schnitt eindringt. Dadurch wird eine wirksame seitliche Führung des Schleif-Bohrwerkzeugs an der Bearbeitungsstelle erreicht.

Das in Fig. 2 dargestellte Ausführungsbeispiel unterscheidet sich von dem Ausführungsbeispiel nach Fig. 1 nur dadurch, daß die Stirnfläche 4' des Schleifkopfes 3 als eine innere ringförmige Hohlkegelstumpf-Fläche ausgeführt ist, die an ihrem äußeren Rand 4'b einen scharfkantigen spitzen Winkel mit der äußeren Seitenwand 6 des Schleifkopfes 3 bildet.

Abweichend hiervon ist beim Ausführungsbeispiel nach Fig. 3 die Stirnfläche 4'' als eine äußere ringförmige Kegelstumpffläche ausgeführt, die an ihrem inneren Rand 4''a einen scharfkantigen spitzen Winkel mit der inneren Seitenwand 5 des Schleifkopfes 3 bildet. Es versteht sich, daß bei den Ausführungen nach den Fig. 2 und 3 jeweils der scharfkantig ausgebildete Rand 4'b bzw. 4''a die Führung des Schleif-Bohrwerkzeugs im Knochen- bzw. Knorpelmaterial übernimmt.

Wie man aus der Zeichnung erkennt, weist der Schleifkopf 3 an seinem der Stirnfläche 4, 4' bzw. 4'' abgekehrten rückwärtigen Ende gegenüber der Innenwand 1a und der Außenwand 1b des hohlzylindrischen Werkzeugschaftes 1 jeweils einen Überstand 7 auf, der mindestens etwa 10 % der Wanddicke des Werkzeugschaftes 1 beträgt. Dieser Überstand 7 verhindert, daß beim Eindringen des Schleifkopfes 3 in das abzutragende Material eine spürbare Berührung mit dem Werkzeugschaft 1 stattfindet. Dadurch wird eine Abbremsung des Werkzeugs und eine unerwünschte Wärmeentwicklung durch Reibung vermieden.

Fig. 4 zeigt ein Schleif-Bohrwerkzeug ähnlich der Fig. 1, wobei jedoch mindestens zwei einander gegenüberliegende Kühlmittelschlitze 8 vorgesehen sind, die sich von der Stirnfläche 4 nach hinten erstrecken, die von der inneren Seitenwand 5 zur äußeren Seitenwand 6 des Schleifkopfes 3 durchgehen. Da das Kühlmittel im Inneren des Werkzeugschaftes 1 zugeführt wird und da zwischen der Stirnfläche 4 und der zu bearbeitenden Fläche eine weitgehende Abdichtung erfolgt, ermöglichen die Kühlmittelschlitze 8 eine Kühlmittelströmung, so daß eine ausreichende Kühlwirkung im Bearbeitungsbereich und insbesondere auch ein Abtransport des abgetragenen Materials sichergestellt sind.

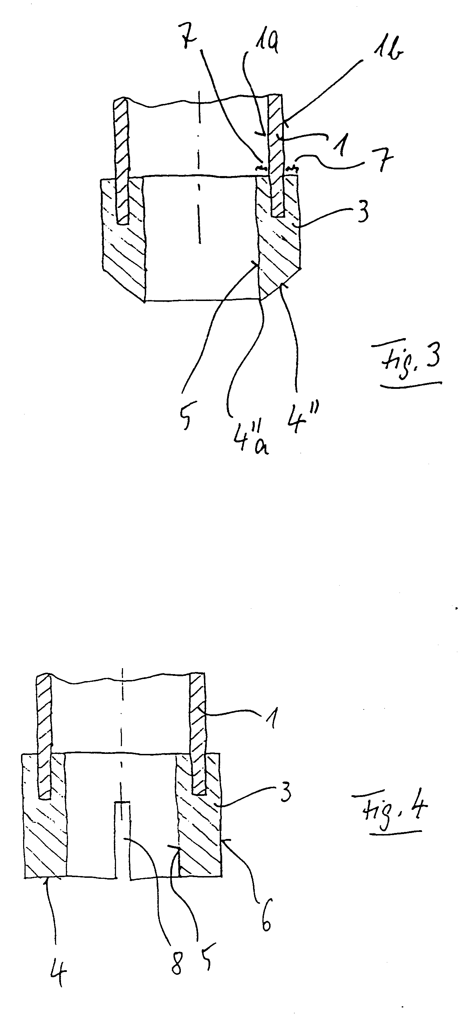

Anstelle der in Fig. 4 dargestellten Kühlmittelschlitze 8 können auch radial durchgehende Kühlmittelbohrungen 9 im Schleifkopf 3 vorgesehen sein, die in Fig. 5 dargestellt sind.

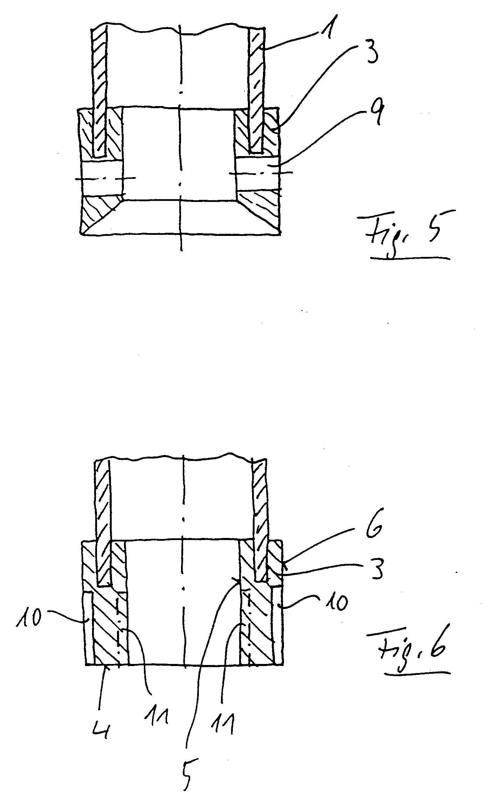

Andere Möglichkeiten zur Unterstützung der Kühlmittelströmung sind in Fig. 6 schematisch angedeutet. Hierbei sind Kühlmittelnuten 10 bzw. 11 in der inneren Seitenwand 5 bzw. der äußeren Seitenwand 6 des Schleifkopfes 3 vorgesehen.

## Patentansprüche

1. Schleif-Bohrwerkzeug zur Gewinnung von Knochenmaterial durch rotierendes Fräsen, bestehend aus einem hohlzylindrischen Werkzeugschaft (1), der an seinem vorderen Ende einen ringförmigen, an seiner Oberfläche mit Schleifkornmaterial besetzten Schleifkopf (3) trägt, dadurch gekennzeichnet, daß die Stirnfläche (4, 4', 4'') des Schleifkopfes (3) an ihrem inneren (4a, 4''a) und/oder äußeren Rand (4b, 4'b) scharfkantig an die benachbarte innere bzw. äußere zylindrische Seitenwand (5 bzw. 6) des Schleifkopfes (3) anschließt.

2. Schleif-Bohrwerkzeug nach Anspruch 1, dadurch gekennzeichnet, daß die Stirnfläche (4) des Schleifkopfes (3) eine senkrecht zur Werkzeuglängsachse (2a) verlaufende Kreisringfläche ist.

3. Schleif-Bohrwerkzeug nach Anspruch 2, dadurch gekennzeichnet, daß die Stirnfläche (4) an ihren beiden Rändern (4a, 4b) scharfkantig ausgeführt ist.

4. Schleif-Bohrwerkzeug nach Anspruch 1, dadurch gekennzeichnet, daß die Stirnfläche (4'') des Schleifkopfes (3) eine äußere ringförmige Kegelstumpffläche ist, die an ihrem inneren Rand (4''a) einen scharfkantigen spitzen Winkel mit der inneren Seitenwand (5) des Schleifkopfes (3) bildet.

5. Schleif-Bohrwerkzeug nach Anspruch 1, dadurch gekennzeichnet, daß die Stirnfläche (4') des Schleifkopfes (3) eine innere ringförmige Hohlkegelstumpf-Fläche ist, die an ihrem äußeren Rand (4'b) einen scharfkantigen spitzen Winkel mit der äußeren Seitenwand (6) des Schleifkopfes (3) bildet.

6. Schleif-Bohrwerkzeug nach Anspruch 1, dadurch gekennzeichnet, daß der Schleifkopf (3) an seinem der Stirnfläche (4, 4', 4'') abgekehrten rückwärtigen Ende gegenüber der Innenwand (5) und der Außenwand (6) des hohlzylindrischen Werkzeugschaftes (1) jeweils einen Überstand (7) von mindestens etwa 10 % der Wanddicke des Werkzeugschaftes (1) aufweist.

7. Schleif-Bohrwerkzeug nach Anspruch 1, dadurch gekennzeichnet, daß sich Kühlmittelschlitze (8) oder Kühlmittelnuten (10, 11) von der Stirnfläche (4) des Schleifkopfes (3) nach hinten erstrecken.

8. Schleif-Bohrwerkzeug nach Anspruch 7, dadurch gekennzeichnet, daß die Kühlmittelschlitze (8) von der inneren Seitenwand (5) zur äußeren Seitenwand (6) des Schleifkopfes (3) durchgehen.

9. Schleif-Bohrwerkzeug nach Anspruch 1, dadurch gekennzeichnet, daß der Schleifkopf (3) radial durchgehende Kühlmittelbohrungen (9) aufweist.

## Claims

1. Drilling and abrasion tool for obtaining bone material by rotary milling, consisting of a hollow-cylindrical tool shank (1) which has, at its front end, an annular abrasion head (3) equipped with abrasive grain material on its surface, characterized in that the inner margin (4a, 4''a) and/or the outer margin (4b, 4'b) of the end face (4, 4', 4'') of the abrasion head (3) adjoin(s) the adjacent inner and/or outer cylindrical side wall (5 and 6, respectively) of the abrasion head via a sharp edge.

2. Drilling and abrasion tool according to Claim 1, characterized in that the end face (4) of the abrasion head (3) is an annular surface extending perpendicular to the longitudinal axis (2a) of the tool.

3. Drilling and abrasion tool according to Claim 2, characterized in that the end face (4) is designed with a sharp edge at its two margins (4a, 4b).

4. Drilling and abrasion tool according to Claim 1, characterized in that the end face (4'') of the abrasion head (3) is an outer annular truncated cone surface which, at its inner margin (4''a), forms a sharp-edged acute angle with the inner side wall (5) of the abrasion head (3).

5. Drilling and abrasion tool according to Claim 1, characterized in that the end face (4') of the abrasion head (3) is an inner annular hollow truncated cone surface which, at its outer margin (4'b), forms a sharp-edged acute angle with the outer side wall (6) of the abrasion head (3).

6. Drilling and abrasion tool according to Claim 1, characterized in that the abrasion head (3), at its rear end facing away from the end face (4, 4', 4''), has, with respect to the inner wall (5) and the outer wall (6) of the hollow-cylindrical tool shank (1), in each case a protrusion (7) of at least approximately 10% of the wall thickness of the tool shank (1).

7. Drilling and abrasion tool according to Claim 1, characterized in that coolant slots (8) or coolant grooves (10, 11) extend rearwards from the end face (4) of the abrasion head (3).

8. Drilling and abrasion tool according to Claim 7, characterized in that the coolant slots (8) run through from the inner side wall (5) to the outer side wall (6) of the abrasion head (3).

9. Drilling and abrasion tool according to Claim 1, characterized in that the abrasion head (3) has radially continuous coolant bores (9).

## Revendications

1. Outil de meulage et de percement pour l'obtention d'une matière osseuse par fraisage rotatif, constitué d'une tige d'outil en cylindre creux (1), qui porte à son extrémité antérieure une tête de meulage en forme d'anneau (3), recouverte à sa surface d'une matière de meulage granulaire, caractérisé en ce que la surface frontale (4, 4', 4'') de la tête de meulage (3) se raccorde à son bord interne (4a, 4''a) et/ou à son bord externe (4b, 4'b) avec une arête vive à la paroi latérale cylindrique interne ou externe voisine (5 ou 6) de la tête de meulage (3).

2. Outil de meulage et de percement selon la revendication 1, caractérisé en ce que la surface frontale (4) de la tête de meulage (3) est une surface en couronne courant perpendiculairement à l'axe longitudinal de l'outil (2a).

3. Outil de meulage et de percement selon la revendication 2, caractérisé en ce que la surface frontale (4) est réalisée avec un arête vive sur ses deux bords (4a, 4b).

4. Outil de meulage et de percement selon la revendication 1, caractérisé en ce que la surface latérale (4'') de la tête de meulage (3) est une surface tronconique en forme d'anneau externe, qui forme à son bord intérieur (4''a) un angle aigu à arête vive avec la paroi latérale interne (5) de la tête de meulage (3).

5. Outil de meulage et de percement selon la revendication 1, caractérisé en ce que la surface frontale (4') de la tête de meulage (3) est une surface tronconique creuse en forme d'anneau interne, qui forme à son bord extérieur (4'b) un angle aigu à arête vive avec la paroi latérale externe (6) de la tête de meulage (3).

6. Outil de meulage et de percement selon la revendication 1, caractérisé en ce que la tête de meulage (3) présente à son extrémité arrière se détournant de la surface frontale (4, 4', 4'') vis-à-vis de la paroi interne (5) et de la paroi externe (6) de la tige d'outil cylindrique creuse (1) à chaque fois une saillie (7) d'au moins environ 10% de l'épaisseur de la paroi de la tige de l'outil (1).

7. Outil de meulage et de percement selon la revendication 1, caractérisé en ce que des fentes (8) ou des rainures (10, 11) pour produit de refroidissement s'étendent à partir de la surface frontale (4) de la tête de meulage (3) vers l'arrière.

8. Outil de meulage et de percement selon la revendication 7, caractérisé en ce que les fentes (8) pour produit de refroidissement courent de la paroi latérale interne (5) à la paroi latérale externe (6) de la tête de meulage (3).

9. Outil de meulage et de percement selon la revendication 1, caractérisé en ce que la tête de meulage (3) présente des percements (9) pour produit de refroidissement s'étendant radialement.
